(19) Europäisches Patentamt
European Patent Office
Office européen des brevets

(11) Publication number: **0 410 025 A1**

# (12) EUROPEAN PATENT APPLICATION

(21) Application number: **89113604.6**

(22) Date of filing: **24.07.89**

(51) Int. Cl.5: **C07D 471/20**, //(C07D471/20, 221:00,209:00,209:00)

(43) Date of publication of application: **30.01.91 Bulletin 91/05**

(84) Designated Contracting States: **BE CH DE ES FR IT LI**

(71) Applicant: **Kawaken Fine Chemicals Co., Ltd.**
**3-3, Nihonbashi Horidome-cho 2-chome**
**Chuo-ku Tokyo(JP)**

(72) Inventor: **Takano, Seiichi**
**1-16-4, Kamo**
**Sendai-shi Miyagi(JP)**
Inventor: **Ogasawara, Kunio**
**2-37-32, Asahigaoka**
**Sendai-shi Miyagi(JP)**
Inventor: **Satoh, Shigeki**
**1532-153, Maze**
**Tsukuba-shi Ibaraki(JP)**
Inventor: **Umezawa, Hiroshi**
**18-7, Koei-cho**
**Kawagoe-shi Saitama(JP)**
Inventor: **Okura, Kazuhiro**
**6-14-24, Arajuku**
**Kawagoe-shi Saitama(JP)**

(74) Representative: **Patentanwälte Viering & Jentschuraura**
**Steinsdorfstrasse 6**
**D-8000 München 22(DE)**

## (54) Method of recemizing optical active vincadifformine compound.

(57) An optical active vincadifformine compound is racemized by heating a member of compounds of the formulae (I) and (II):

(I)

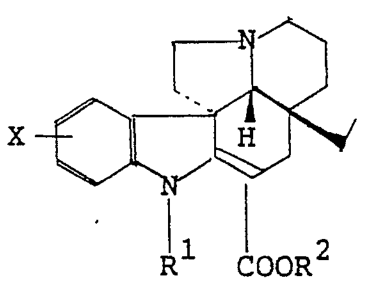

and

(II)

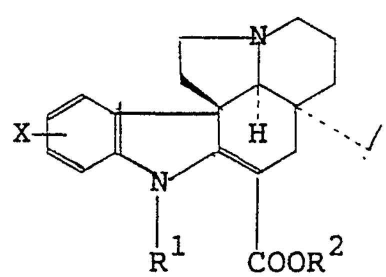

wherein X represents a member selected from the group consisting of a hydrogen atom, halogen atoms, lower alkyl radicals and lower alkyloxy radicals, $R^1$ represents a member selected from the group consisting of a hydrogen atom and lower alkyl radicals, and $R^2$ represents a member selected from the group consisting of lower alkyl radicals having 1 to 5 carbon atoms and a benzyl radical, at a temperature of 100 °C or more preferably in an organic solvent to prepare a racemic modification containing an aimed optical antipode compound.

EP 0 410 025 A1

# METHOD OF RACEMIZING OPTICAL ACTIVE VINCADIFFORMINE COMPOUND

## BACKGROUND OF THE INVENTION

### 1. Field of the Invention

The present invention relates to a method of racemizing an optical active vincadifformine compound. More particularly, the present invention relates to a method of racemizing a practically useless optical active vincadifformine compound to produce a racemic modification thereof containing an optical antipode vincadifformine compound which is useful as a precursor of another indole alkaloid having a pharmaceutical activity, for example, vincamine, apovincamine, eburumamonine, and 11-bromovincamine, which are useful as a cerebral vasodilator.

### 2. Description of the Related Arts

Vincadifformine compounds are a group of natural indole alkaloids and usually are isolated as useful optical active compounds having a pharmaceutical activity by extraction from a natural source.

The vincadifformine compound obtained from a natural source, however, is disadvantageous in that the quality of the resultant product is not stable and the quantity of the product is restricted, and thus it is difficult to supply a vincadifformine product having a satisfactory quality in sufficient amounts to meet the current demands of the pharmaceutical industry. Under the above circumstance, a technology of producing the vincadifformine compounds by an organic synthesis has been developed to ensure a supply thereof with a satisfactory uniform quality , and in a large amount.

For example, various methods of synthesizing the vincadifformine compounds are disclosed in U.S. Patent No. 4,220,774 and Japanese Unexamined Patent Publication (Kokai) Nos. 63-54372 and 1-135786.

Nevertheless, the synthetic production of the vincadifformine compounds is disadvantageous in that the resultant product of the organic synthesis is in the form of a racemic mixture, and thus a necessary optical antipode must be isolated from an unnecessary optical antipode, by optical resolution, but in this optical resolution of the racemic mixture the unnecessary optical antipode vincadifformine compound always remains.

If the unnecessary compound can be converted to a racemic mixture containing the necessary optical antipode compound, it would be possible to isolate the necessary compound from the racemic mixture by optical resolution, and to utilize the isolated compound for practical use, but a method of racemizing an optical active vincadifformine compound was not known until the present invention.

## SUMMARY OF THE INVENTION

An object of the present invention is to provide a method of racemizing an optical active vincadifformine compound at a high efficiency and a low cost.

The above object is realized by the method of the present invention of racemizing an optical active vincadifformine compound, which method comprises heating a member of optical active vincadifformine compounds of the formulae (I) and (II).

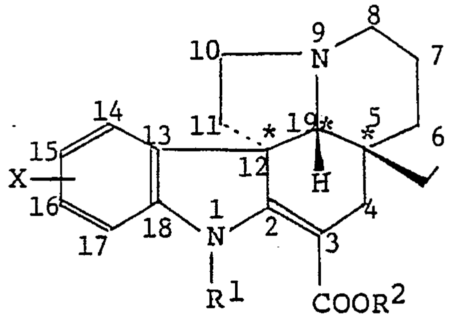

(I) . and

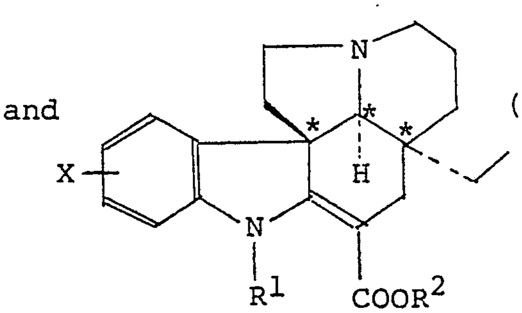

(II)

wherein X represents a member selected from the group consisting of a hydrogen atom, halogen atoms, lower alkyl radicals preferably having 1 to 3 carbon atoms, and lower alkyloxy radicals preferably having 1 to 3 carbon atoms, $R^1$ represents a member selected from the group consisting of a hydrogen atom and lower alkyl radicals preferably having 1 to 3 carbon atoms, and $R^2$ represents a member selected from the group consisting of lower alkyl radicals having 1 to 5 carbon atoms and a benzyl radical, at a temperature of 100°C or more.

## DESCRIPTION OF THE PREFERRED EMBODIMENTS

The method of the present invention can be applied to all the dextro and levo rotatory optical active vincadifformine compounds of the formulae (I) and (II). For example, the vincadifformine compounds to be racemized in accordance with the method of the present invention include lower alkyl esters and benzyl esters of (+) and (-)-vincadifformines, (+) and (-)-15-bromovincadifformines, (+) and (-)-16-bromovincadifformines, (+) and (-)-16-methoxyvincadifformines, and (+) and (-)-minovines.

In the method of the present invention, the racemization of the vincadifformine compound can be effected in the absence of a solvent medium, but preferably the racemization of the vincadifformine compound is carried out in an organic solvent. The organic solvent is preferably inert and not reactive with the vincadifformine compound. The inert organic solvent is effective for controlling the heating temperature to a predetermined level.

The organic solvent preferably comprises at least one non-protonic polar solvent which can be selected from, for example, the group consisting of aromatic hydrocarbons, for example, xylene, ethylbenzene, cumene, and butylbenzene; halogenated aromatic hydrocarbons monochlorobenzene and dichlorobenzenes; acyclic amide compounds, for example, dimethylformamide, dimethylacetamide and tetramethyl urea; cylic amide compounds, for example, N-methylpyrrolidinone and 1,3-dimethyl-2-imidazolidinone; dimethylaniline; methoxybenzene; and lower alkyl esters, for example, methyl and ethyl esters, of hexanic acid, malonic acid, and benzoic acid.

The organic solvent may contain a protonic solvent, for example, water, acetic acid, or an aliphatic alcohol, although this type of solvent results in a lower yield of the racemized vincadifformine compound.

The organic solvent is preferably used in an amount of about 0.5 to about 10 times the weight of the vincadifformine compound to be racemized.

In the method of the present invention, the racemization is carried out by heating the vincadifformine compound at a temperature of 100°C or more. If the heating temperature is lower than 100°C, the racemization reaction rate of the vincadifformine compound is very low and the resultant racemization is unsatisfactory. Preferably, the racemization temperature is from 100°C to 200°C when the racemization is carried out in the ambient open atmosphere. If the racemization temperature is higher than 200°C in the ambient open atmosphere, the vincadifformine compound may be thermally decomposed or decarbonated, and thus the resultant racemization product contains an increased amount of side-reaction products.

When the racemization atmosphere is closed and consists essentially of an inert gas, for example, nitrogen or argon gas, the racemization reaction can be effected at a temperature of from 100°C to 300°C without a decomposition of the vincadifformine compound.

The heating operation is preferably carried out while refluxing the organic solvent under the ambient atmosphere pressure. This mode of heating is advantageous in that the racemization temperature can be simply and easily maintained at a constant value. In another mode, the racemization operation is carried out in a high boiling point organic solvent while controlling the heating temperature to a predetermined temperature lower than the boiling point of the organic solvent. In still another mode, the racemization operation is carried out in a closed inert gas atmosphere under a pressure at a higher temperature than the boiling point of the organic solvent under atmosphere pressure.

The manner of heating is not specifically limited, and thus the racemization solution can be heated by any conventional heating operator. Preferably, however, the racemization solution is heated directly by radiation of a high frequency electron beam thereto. This heating mode is advantageous in that the racemization reaction can be completed within a short time.

In the heating operation in the method of the present invention, preferably the racemization solution is placed in an inert gas atmosphere, to prevent an undesirable decomposition and oxidation of the vincadifformine compound. When the racemization temperature is high, for example, 120°C or more, the inert gas atmosphere must be completely sealed.

After the racemization operation is completed, the organic solvent is removed from the resultant racemized compound solution by distillation under a reduced pressure, and the remaining racemic

modification is crystallized in a crystallizing organic solvent, for example, methyl alcohol or ethyl alcohol, and then recovered. Alternatively, the racemic modification is recovered by dissolving same in a lower aliphatic alcohol, for example, methyl alcohol, ethyl alcohol or isopropyl alcohol, or acetone, or an organic or inorganic acid, for example, oxalic acid, tartaric acid or perchloric acid, is added to the solution to convert the racemic vincadifformine compounds to salts thereof, and the salts are then recrystallized and recovered.

In a further alternative, after the organic solvent is distilled away, the resultant distillation residue comprising the racemic modification is directly refined by a column chromatography and recovered.

In the present method, the racemization yield of the racemic modification varies in accordance with the type of organic solvent and the heating temperature and time. Usually, the racemic modification can be obtained at a yield of 20 to 95%, based on the weight of the starting vincadifformine compound.

The racemic modifications contain desired optical antipode compounds which can be isolated by the optical resolution method disclosed, for example, in Japanese Unexamined Patent Publication No. 1-135,786. In the optical resolution method, racemic vincadifformine compounds are reacted with an optically active tartaric acid to provide two diastereomeric salts of the compounds, and the two diasteromeric salts are separated from each other by an optical resolution due to difference in solubility between the two salts in a specific solvent, for example, methyl alcohol, ethyl alcohol, 1-propyl alcohol, 2-propyl alcohol, and acetone.

## EXAMPLES

The method of the present invention will be further explained by way of specific examples.

### Example 1

A racemization solution was prepared by dissolving 0.5 g of (-)-16-bromovincadifformine having a specific rotatory power $[\alpha]_D$ of -471.4 (C = 1.0, chloroform) in 5 ml of dichlorobenzene, degasified, and then heated at the boiling temperature of dichlorobenzene under atmospheric pressure while refluxing for 14 hours.

After the racemization operation was completed, the racemization solution was treated with a silica gel column chromatograph containing a mixture of 1 part by volume of ethyl ether with 2 parts by volume of hexane. The refined racemic (±)-16-bromovincadifformine was obtained in an amount of 0.39 g corresponding to a yield of 77%. The racemic compound exhibited a specific rotatory power of -1.6. This value shows that the starting (-)-16-bromovincadifformine was substantially quantitatively racemized.

### Example 2

A solution of 5.0 g of the same grade of (-)-16-bromovincadifformine as that described in Example 1 in 45 g of dimethylformamide was placed in a reaction vessel, the inside space of the closed reaction vessel was filled with a nitrogen gas, and the solution was then heated at the boiling point of dimethylformamide under atmospheric pressure for 24 hours while refluxing.

After the racemization operation was completed, the resultant solution was subjected to distillation under a reduced pressure to evaporate away dimethylformamide, the distillation residue was dissolved in 30 ml of isopropyl alcohol, the resultant solution was added 1.8 g of oxalic acid dihydrate, and the whole was cooled with ice water so that the resultant oxalate immediately deposited in the form of solid particles from the solution. The resultant deposit was separated from the solution by a filtration, and dried, and comprised light yellow particles of a racemic (±)-16-bromovincadifformine oxalate in an amount of 5.2 g corresponding to an yield of 85.5%.

A solution of 0.23% by weight of the racemic modification in methyl alcohol exhibited a specific rotatory power of -5.7, and thus it was found that the racemization yield from the starting compound was 97%.

### Examples 3 to 7

In each of Examples 3 to 7, the same procedures as in Example 2 were carried out except that 5 g of (+)-16-bromovincadifformine having a specific rotatory power $[\alpha]_D$ of +282.1 (C = 0.21, Chloroform) was

dissolved in 45 g of the organic solvent indicated in Table 1, and the solution was heated at the temperature and for the time indicated in Table 1.

The oxalate of (+)-16-bromovincadifformine exhibited a specific rotatory power $[\alpha]_D^{23}$ of 176.2 (C=0.23, MeOH).

The results are shown in Table 1.

Table 1

| | Racemization operation | | | Racemization product | | |
|---|---|---|---|---|---|---|
| Example No. | Organic solvent | Temperature (°C) | Time (hr) | Oxalate yield (%) | Specific rotatory power | Racemization yield (%) |
| 3 | Dimethyllaniline | 120 | 74 | 49.0 | +12.0 | 97.5 |
| 4 | Isopropylbenzene | 151 | 72 | 84.2 | +34.2 | 80.6 |
| 5 | t-Butylbenzene | 167 | 40 | 94.8 | +2.3 | 98.7 |
| 6 | Methoxybenzene | 153 | 34 | 88.0 | +17.6 | 90.0 |
| 7 | Dimethylacetamide | 164 | 10 | 82.3 | +3.7 | 97.9 |

Example 8

A solution of 112 mg (0.3 m moles) of (-)-vincadifformine having a specific rotatory power $[\alpha]_D^{27}$ of -646.03 (c=0.60, chloroform) in 2 ml of dimethylformamide was placed in a glass tube having a capacity of 10 ml, the inside of the tube was completely filled with argon gas, and the tube was then sealed.

A high frequency election beam radiation was applied to the tube placed in an electron range (available under a trademark of Electron Range MR-M22, made by Hitachi Seisakusho) at 500 W and 2450 MHz for 20 minutes.

It was assumed that the temperature of the solution in the tube was raised to about 300°C.

After cooling the tube, the solution was subjected to distillation under a reduced pressure to evaporate away the solvent, and the distillation residue was treated with a silica gel column chromatography.

The resultant product was it racemic (±)-vincadifformine in an amount of 88 mg corresponding to an yield of 78%.

This racemic modification exhibited a specific rotatory power $[\alpha]_D^{24}$ of -6.02 (c=1.56, chloroform) and showed a racemization yield of 99%.

Examples 9 and 10

In each of Examples 9 and 10, the same procedures as in Example 8 were carried out except that, in Example 9, the heating time was 15 minutes, and in Example 10, the organic solvent was dimethylacetamide.

The conditions and results of the racemization operation are shown in Table 2.

Table 2

| | Racemization operation | | | Racemization product | |
|---|---|---|---|---|---|
| Example No. | Solvent | Time (min) | Yield (%) | Specific rotatory power $[\alpha]_D$ | Racemization yield (%) |
| 9 | Dimethylformamide | 15 | 76.3 | +39.3 (c-0.87, $CHCl_3$) | 93.9 |
| 10 | Dimethylacetamide | 20 | 16.7 | + 4.7 (c-0.51, $CHCl_3$) | 99.3 |

As the examples clearly indicate, the optical active vincadifformine compounds can be easily converted to racemic modifications thereof at a high yield and at a high racemization yield.

## Claims

1. A method of racemizing an optical active vincadifformine compound, comprising heating a member of optical active vincadifformine compounds of the formulae (I) and (II):

(I)

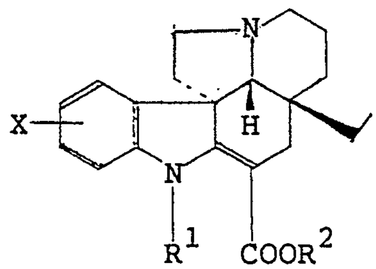

and

(II)

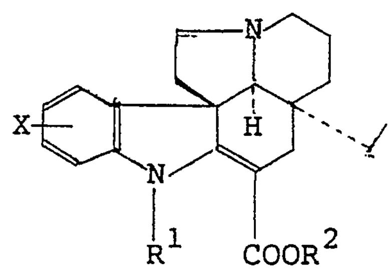

wherein X represents a member selected from the group consisting of a hydrogen atom, halogen atoms, lower alkyl radicals and lower alkyloxy radicals, $R^1$ represents a member selected from the group consisting of a hydrogen atom and lower alkyl radicals, and $R^2$ represents a member selected from the group consisting of lower alkyl radicals having 1 to 5 carbon atoms and a benzyl radical, at a temperature of 100° C or more.

2. The method as claimed in claim 1, wherein the racemization of the vincadifformine compound is carried out in an organic solvent.

3. The method as claimed in claim 2, wherein the organic solvent comprises at least one non-protonic polar liquid compound.

4. The method as claimed in claim 3, wherein the non-protonic polar compound is selected from the group consisting of halogenated aromatic hydrocarbons, aromatic hydrocarbons, dimethylformamide, dimethylacetamide, dimethylaniline, methoxybenzene N-methylpyrrolidinone, tetramethyl urea, 1,3-dimethyl-2-imidazolidinone and lower alkyl esters of hexanic acid, malonic acid and benzoic acid.

5. The method as claimed in claim 1, wherein the heating operation is effected by means of a high frequency electron beam radiation.

6. The method as claimed in claim 1, wherein the heating operation is carried out at a temperature of from 100° C to 200° C.

7. The method as claimed in claim 1, wherein the heating operation is carried out in a closed atmosphere consisting essentially of an inert gas at a temperature of from 100° C to 300° C.

8. The method as claimed in claim 1, wherein the optical active vincadifformine compound is selected from the group consisting of lower alkyl esters and benzyl esters of
(-) and (+)-vincadifformines,
(-) and (+)-15-bromovincadifformines,
(-) and (+)-16-bromovincadifformines,
(-) and (+)-16-methoxyvincadifformines, and
(-) and (+)-minovines

European Patent Office

# EUROPEAN SEARCH REPORT

Application Number

EP 89 11 3604

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol.111, 1989, absatract no. 134611u, Columbus, Ohio, US; TAKANO, SEIICHI et al.: "Racemization of (-)-vincadifformine using a microwave oven", & CHEM. LETTER 1989, (1), 87-8<br>* Abstract *<br>----- | 1-4 | C 07 D 471/20 //<br>(C 07 D 471/20<br>C 07 D 221:00<br>C 07 D 209:00<br>C 07 D 209:00 ) |
| | | | **TECHNICAL FIELDS SEARCHED (Int. Cl.5)** |
| | | | C 07 D 471/00 |

The present search report has been drawn up for all claims

| Place of search | Date of completion of the search | Examiner |
|---|---|---|
| THE HAGUE | 19-03-1990 | VAN BIJLEN H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)